# EUROPÄISCHE PATENTSCHRIFT

(11) **EP 2 844 313 B1**
(45) Veröffentlichungstag und Bekanntmachung des Hinweises auf die Patenterteilung: **01.06.2016**
(21) Anmeldenummer: 13723406.8
(22) Anmeldetag: 26.04.2013
(51) Int. Cl.: A61M 1/16, A61M 1/34

(54) **VORRICHTUNG ZUR DURCHFÜHRUNG EINES VERFAHRENS ZUR KONSERVIERUNG EINER BLUTBEHANDLUNGSVORRICHTUNG SOWIE VERFAHREN ZUR KONSERVIERUNG EINER BLUTBEHANDLUNGSVORRICHTUNG**
DEVICE FOR PERFORMING A METHOD FOR CONSERVING A BLOOD TREATMENT DEVICE AND METHOD FOR CONSERVING A BLOOD TREATMENT DEVICE
DISPOSITIF PERMETTANT DE METTRE EN OEUVRE UN PROCÉDÉ DE CONSERVATION D'UN DISPOSITIF DE TRAITEMENT DU SANG ET PROCÉDÉ DE CONSERVATION D'UN DISPOSITIF DE TRAITEMENT DU SANG

(30) Priorität: 30.04.2012 DE 102012008551; 30.04.2012 US 201261640037 P
(43) Veröffentlichungstag der Anmeldung: 11.03.2015
(73) Patentinhaber: Fresenius Medical Care Deutschland GmbH, 61352 Bad Homburg (DE)
(72) Erfinder: WIESEN, Gerhard, 61352 Bad Homburg v.d.H. (DE)
(74) Vertreter: Oppermann, Frank
(86) Internationale Anmeldenummer: PCT/EP2013/001265
(87) Internationale Veröffentlichungsnummer: WO 2013/164079

(56) Entgegenhaltungen:
- FR-A1- 2 700 121
- US-A- 5 895 578
- US-B1- 6 187 207

## Beschreibung

Die Erfindung betrifft eine Vorrichtung zur Durchführung eines Verfahrens zur Konservierung einer extrakorporalen Blutbehandlungsvorrichtung sowie ein Verfahren zur Konservierung einer extrakorporalen Blutbehandlungsvorrichtung. Darüber hinaus betrifft die Erfindung eine extrakorporale Blutbehandlungsvorrichtung mit einer Vorrichtung zur Durchführung eines Verfahrens zur Konservierung der Blutbehandlungsvorrichtung.

Die bekannten Dialysevorrichtungen verfügen über einen extrakorporalen Blutkreislauf und ein Dialysierflüssigkeitssystem. Das Dialysierflüssigkeitssystem der bekannten Dialysevorrichtungen umfasst eine Dialysierflüssigkeitszuführleitung, die von einer Dialysierflüssigkeitsquelle zu der Dialysierflüssigkeitskammer eines Dialysators führt, und eine Dialysierflüssigkeitsabführleitung, die von der Dialysierflüssigkeitskammer des Dialysators zu einem Ablauf führt. Während die Dialysierflüssigkeit die Dialysierflüssigkeitskammer des Dialysators durchströmt, findet ein Stofftransport über die Membran des Dialysators in die Blutkammer statt. Bei der sogenannten Hämo(dia)filtration wird ein Teil der durch die Membran des Dialysators abgezogenen Flüssigkeit durch eine sterile Substitutionsflüssigkeit (Substituat) ersetzt, die entweder stromauf oder stromab des Dialysators dem extrakorporalen Blutkreislauf zugeführt wird. Die Zuführung des Substituats erfolgt über eine Substituatleitung, die zu dem extrakorporalen Blutkreislauf führt.

In der Praxis hat sich gezeigt, dass sich das Substituat während der Blutbehandlung online steril aus der Dialysierflüssigkeit gewinnen lässt.

Es sind Dialysevorrichtungen bekannt, bei denen das Substituat während der Dialysebehandlung (online) aus der Dialysierflüssigkeit gewonnen wird. Um sicherzustellen, dass das online gewonnene Substituat steril und pyrogenfrei ist, wird die Dialysierflüssigkeit durch einen Sterilfilter geleitet, der durch eine Keime zurückhaltende Membran in zwei Kammern unterteilt ist. Eine Dialysevorrichtung mit einem Sterilfilter zur Gewinnung von Substituat ist beispielsweise aus der US 6,187,207 B1 bekannt.

Zur Reinigung und Desinfektion von Blutbehandlungsvorrichtungen sind verschiedene Verfahren bekannt. Vor der Inbetriebnahme wird die Blutbehandlungsvorrichtung mit einer Flüssigkeit gespült, die ein Reinigungs- und/oder Desinfektionsmittel enthält. Dadurch lassen sich auch hartnäckige Verschmutzungen, wie Biofilm, Algen, Eiweißablagerungen und Blutrückstände sicher und rasch entfernen.

Einen Sonderfall stellt die Lagerung einer Blutbehandlungsvorrichtung über einen längeren Zeitraum, insbesondere vor der ersten Inbetriebnahme dar. In diesem Zeitraum stellt sich nicht nur das Problem, Verunreinigungen auszuschließen, sondern auch die Blutbehandlungsvorrichtung vor Frost zu schützen. Zur Konservierung werden daher die Flüssigkeitssysteme der bekannten Blutbehandlungsvorrichtungen im Allgemeinen mit einem Frostschutzmittel befüllt.

Der Erfindung liegt die Aufgabe zu Grunde, ein Verfahren anzugeben, dass eine Konservierung einer extrakorporalen Blutbehandlungsvorrichtung erlaubt, ohne dass die Gefahr der Bildung von Verunreinigungen besteht, die sich nicht sicher und rasch mit den bekannten Reinigungs- und Desinfektionsmitteln entfernen ließen.

Darüber hinaus ist eine Aufgabe der Erfindung, eine Vorrichtung bereitzustellen, mit der sich das Verfahren zur Konservierung der Blutbehandlungsvorrichtung einfach und sicher durchführen lässt.

Eine weitere Aufgabe der Erfindung liegt darin, eine Blutbehandlungsvorrichtung mit einer Vorrichtung zur Durchführung des Konservierungsverfahrens zu schaffen.

Die Lösung dieser Aufgaben erfolgt mit den Merkmalen der unabhängigen Patentansprüche. Vorteilhafte Ausführungsformen der Erfindungen sind Gegenstand der Unteransprüche.

Verschiedene Untersuchungen des Erfinders über die Ursachen bakterieller Verunreinigungen in Dialysierflüssigkeitssystemen haben gezeigt, dass eine erhöhte Gefahr von Verunreinigungen dann bestehen kann, wenn das Dialysierflüssigkeitssystem zur Konservierung der Blutbehandlungsvorrichtung mit einem Frostschutzmittel befüllt werden sollte.

Das erfindungsgemäße Verfahren zur Konservierung einer Blutbehandlungsvorrichtung verringert die Gefahr von Verunreinigungen, wenn das Dialysierflüssigkeitssystem der extrakorporalen Blutbehandlungsvorrichtung mit einem Frostschutzmittel befüllt werden sollte. Es beruht darauf, nur den Teil des Dialysierflüssigkeitssystems mit einem Frostschutzmittel zu befüllen, der das Volumen einschließt, das stromauf des Sterilfilters liegt, mit dem steriles Substituat aus der Dialysierflüssigkeit gewonnen wird. Demnach wird der Teil des Dialysierflüssigkeitssystems, der das Volumen einschließt, das stromab des Sterilfilters liegt, nicht mit einem Frostschutzmittel befüllt. Dieser Teil des Dialysierflüssigkeitssystems wird nachfolgend als Substituatsstrecke bezeichnet. Dabei werden die beiden Volumina, die mit Flüssigkeit gefüllt oder nicht mit Flüssigkeit gefüllt sind, von der Membran getrennt, die den Sterilfilter in zwei Kammern unterteilt.

Im Allgemeinen befindet sich in der Substituatsstrecke eine Flüssigkeit, die ein Reinigungs- und Desinfektionsmittel enthält. Mit einer derartigen Flüssigkeit wird das Dialysierflüssigkeitssystem der Blutbehandlungsvorrichtungen nach der ersten Inbetriebnahme gefüllt. Diese Flüssigkeit wird nach dem erfindungsgemäßen Verfahren abgelassen. Somit bleibt nur der übrige Teil des Dialysierflüssigkeitssystems befüllt.

Die erfindungsgemäße Vorrichtung zur Durchführung des erfindungsgemäßen Verfahrens ermöglicht es, nach dem Entfernen des Sterilfilters auf einfache und sichere Weise die Substituatstrecke von dem übrigen Teil des Dialysierflüssigkeitssystems abzutrennen und in der Substituatstrecke befindliche Flüssigkeit abzulassen. Die Flüssigkeit in der Substituatsstrecke kann beispielsweise abgepumpt werden. Hierzu kann die im Dialysierflüssigkeitssystem der bekannten Blutbehandlungsvorrichtung vorhandene Dialysierflüssigkeitspumpe in Betrieb gesetzt werden.

Die in der Praxis eingesetzten Sterilfilter verfügen im Allgemeinen über einen Einlass und einen Auslass für die erste Kammer und einen Einlass und einen Auslass für die zweite Kammer, um beide Kammern im Durchfluss betreiben zu können. Wenn die bekannten Sterilfilter zur Gewinnung eines sterilen Substituats verwendet werden, wird die erste Kammer von Dialysierflüssigkeit durchströmt, während das Substituat aus der zweiten Kammer abgezogen wird. Bei Sterilfiltern, deren erste und zweite Kammer jeweils einen Einlass und einen Auslass aufweisen, müssen Einlass und Auslass beider Kammern an die Blutbehandlungsvorrichtung angeschlossen werden. Folglich verfügt die Blutbehandlungsvorrichtung über vier geräteseitige Anschlussstücke und der Sterilfilter über vier filterseitigen Anschlussstücke. Es ist aber auch möglich, dass die zweite Kammer des zur Gewinnung von Substituat verwendeten Sterilfilters nur einen Auslass aufweist. Dann braucht die Blutbehandlungsvorrichtung nur über drei geräteseitige Anschlussstücke und der Sterilfilter nur über drei filterseitige Anschlussstücke zu verfügen. Die Erfindung sieht daher zwei alternative Ausführungsformen vor.

Bei der einen alternativen Ausführungsform verfügt die erfindungsgemäße Vorrichtung über vier Anschlussstücke, wobei das erste und zweite Anschlussstück mit einer ersten Verbindungsleitung und das dritte und vierte Anschlussstück mit einer zweiten Verbindungsleitung miteinander verbunden sind. Mit der erfindungsgemäßen Vorrichtung kann eine erste Strömungsverbindung mit den beiden Anschlussstücken hergestellt werden, an denen Ein- und Auslass der einen Kammer des Sterilfilters angeschlossen werden, und eine Strömungsverbindung mit den beiden Anschlussstücken hergestellt werden, an denen Ein- und Auslass der zweiten Kammer des Sterilfilters angeschlossen werden. Dadurch wird der Teil des Dialysierflüssigkeitssystems, der die Substituatsstrecke umfasst, von dem übrigen Teil des Dialysierflüssigkeitssystems getrennt. Unter Anschlussstücken werden alle Mittel zur Herstellung einer Verbindung oder Strömungsverbindung verstanden, wobei mehrere Anschlussstücke auch eine Einheit bilden können.

Bei der anderen alternativen Ausführungsform ist das erste und zweite Anschlussstück mit einer ersten Verbindungsleitung miteinander verbunden, während das dritte Anschlussstück steril verschlossen ist, um den Teil des Dialysierflüssigkeitssystems, der die Substituatsstrecke umfasst, von dem übrigen Teil des Dialysierflüssigkeitssystems zu trennen. Das dritte Anschlussstück kann in der Art einer Verschlusskappe ausgebildet sein, die das geräteseitige Anschlussstück steril verschließt, wenn die erfindungsgemäße Vorrichtung an die Blutbehandlungsvorrichtung angeschlossen ist.

Um in der Substituatsstrecke befindliche Flüssigkeit ablassen zu können, verfügt die erfindungsgemäße Vorrichtung über eine Entlüftungseinrichtung. Die Entlüftungseinrichtung ist vorzugsweise wieder ein Sterilfilter, über den Luft in das von der Substituatsstrecke eingeschlossene Volumen beim Ablassen der Flüssigkeit zuströmen kann.

Die erfindungsgemäße Vorrichtung kann unterschiedlich ausgebildet sein. Allein entscheidend ist, dass die genannten Strömungsverbindungen zwischen den geräteseitigen Anschlussstücken der Blutbehandlungsvorrichtung hergestellt werden können. Von Vorteil ist, wenn die erfindungsgemäße Vorrichtung in der Art eines Adapters ausgebildet ist, der sich einfach und sicher mit den geräteseitigen Anschlüssen verbinden lässt, wenn der Sterilfilter von der Blutbehandlungsvorrichtung abgenommen ist. Die Verbindungsleitungen zwischen den Anschlussstücken, können feste oder flexible Leitungen sein. Sie können als Kanäle in festen Körpern ausgebildet oder Schlauchleitungen sein.

Die bekannten Blutbehandlungsvorrichtungen verfügen über Halterungen zur Befestigung der Sterilfilter. Vorzugsweise ist die erfindungsgemäße Vorrichtung derart ausgebildet, dass sie anstelle der Sterilfilter passend in die Halterungen der Blutbehandlungsvorrichtungen eingesetzt werden kann.

Die geräteseitigen Anschlussstücke und die Anschlussstücke der erfindungsgemäßen Vorrichtung können als Stecker oder Buchsen ausgebildet sein. Vorzugsweise bilden jeweils zwei Anschlussstücke der erfindungsgemäßen Vorrichtung einen gemeinsamen Stecker, der passend in eine geräteseitige Buchse der Blutbehandlungsvorrichtung eingesteckt werden kann, an der ansonsten die jeweiligen Anschlussstücke des Sterilfilters angeschlossen werden.

Im Folgenden wird ein Ausführungsbeispiel der Erfindung unter Bezugnahme auf die Zeichnungen näher erläutert.

Es zeigen:
- Fig. 1: die wesentlichen Komponenten einer extrakorporalen Blutbehandlungsvorrichtung, wobei zur Gewinnung eines sterilen Substituats aus der Dialysierflüssigkeit ein Sterilfilter an die Blutbehandlungsvorrichtung angeschlossen ist,
- Fig. 2: die extrakorporale Blutbehandlungsvorrichtung von Fig. 1, wobei zur Durchführung eines Verfahrens zur Konservierung der Blutbehandlungsvorrichtung die erfindungsgemäße Vorrichtung an die Blutbehandlungsvorrichtung angeschlossen ist,
- Fig. 3: eine Teilansicht einer alternativen Ausführungsform der Blutbehandlungsvorrichtung von Fig. 1, wobei zur Gewinnung eines sterilen Substituats aus der Dialysierflüssigkeit ein alternative Ausführungsform des Sterilfilters an die Blutbehandlungsvorrichtung angeschlossen ist und
- Fig. 4: die Blutbehandlungsvorrichtung von Fig. 3, wobei zur Gewinnung eines sterilen Substituats aus der Dialysierflüssigkeit eine alternative Ausführungsform der erfindungsgemäßen Vorrichtung an die Blutbehandlungsvorrichtung angeschlossen ist.

Die Blutbehandlungsvorrichtung, insbesondere Hämo(dia)filtrationsvorrichtung weist einen Dialysator 1 auf, der durch eine Membran 2 in eine von Dialysierflüssigkeit durchflossene Dialysierflüssigkeitskammer 3 und eine vom Blut durchflossene Blutkammer 4 getrennt ist.

Zu der Blutkammer 4 führt eine Blutzuführleitung 5, in die eine Blutpumpe 6 geschaltet ist, während von der Blutkammer 4 eine Blutrückführleitung 7 abgeht. Die Blutzuführ- und -abfuhrleitung 5, 7 bilden zusammen mit der Blutkammer 4 den extrakorporalen Blutkreislauf I der Blutbehandlungsvorrichtung.

Nachfolgend wird das Dialysierflüssigkeitssystem II der Blutbehandlungsvorrichtung beschrieben. Das Dialysierflüssigkeitssystem II weist eine Dialysierflüssigkeitszuführleitung 8, die von einer Dialysierflüssigkeitsquelle 9 zu der Dialysierflüssigkeitskammer 3 führt, und eine Dialysierflüssigkeitsrückführleitung 10 auf, die von der Dialysierflüssigkeitskammer 3 abgeht und zu einem Ablauf 11 führt. Die Dialysierflüssigkeitszuführleitung 8 weist einen ersten Abschnitt 8A auf, der von der Dialysierflüssigkeitsquelle 9 zu der ersten Kammer 12A eines ersten Sterilfilters 12 führt. In den ersten Abschnitt 8A der Dialysierflüssigkeitszuführleitung 8 ist die eine Kammer 13A einer Bilanziereinrichtung 13 geschaltet. Von der zweiten Kammer 12B des ersten Sterilfilters 12 geht der zweite Abschnitt 8B der Dialysierflüssigkeitszuführleitung 8 ab, der zu der Dialysierflüssigkeitskammer 3 führt.

Die Dialysierflüssigkeitsrückführleitung 10 teilt sich in zwei Abschnitte 10A und 10B auf, die zu dem Ablauf 11 führen. In dem ersten Abschnitt 10A ist eine Dialysierflüssigkeitspumpe 14 geschaltet, während in dem zweiten Abschnitt 10B eine Ultrafiltratpumpe 26 geschaltet ist. In den ersten Abschnitt 10A ist darüber hinaus die andere Kammer 13B der Bilanziereinrichtung 13 geschaltet.

Zur Gewinnung eines Substituats aus der Dialysierflüssigkeit verfügt die Hämo(dia)filtrationsvorrichtung über einen zweiten Sterilfilter 15, der über eine semipermeable Membran 15C in eine erste Kammer 15A und eine zweite Kammer 15B unterteilt ist. Der zweite Sterilfilter 15 bildet eine austauschbare Einheit, die an die Blutbehandlungsvorrichtung angeschlossen oder von der Behandlungsvorrichtung abgenommen werden kann.

Zum Anschluss und zur Befestigung des zweiten Sterilfilters 15 verfügt die Blutbehandlungsvorrichtung über eine Halterung 16, die ein erstes Anschlussstück 16A, ein zweites Anschlussstück 16B, ein drittes Anschlussstück 16C und ein viertes Anschlussstück 16D aufweist. Die geräteseitigen Anschlussstücke 16A bis 16D können als Buchsen ausgebildet sein.

Der zweite Sterilfilter 15 weist ein erstes Anschlussstück 15D, ein zweites Anschlussstück 15E, ein drittes Anschlussstück 15F und ein viertes Anschlussstück 15G auf. Die filterseitigen Anschlussstücke 15D bis 15G können passend mit den geräteseitigen Anschlussstücken 16A bis 16D verbunden werden. Die filterseitigen Anschlussstücke können entsprechende Stecker sein.

Das dritte und vierte geräteseitige Anschlussstück 16C, 16D sind mit einer Verbindungsleitung 17A miteinander verbunden, an der eine Bypassleitung 17B angeschlossen ist. An der Bypassleitung 17B befinden sich zwei Absperrorgane 18A und 18B. Zwischen den beiden Absperrorganen 18A und 18B zweigt von einem SubstituatPort 17C eine Substituatleitung 17D ab, in die eine Substituatpumpe 19 geschaltet ist. Die Substituatleitung 17D führt zu dem extrakorporalen Blutkreislauf I stromauf oder stromab der Blutkammer 4, um Substituat dem extrakorporalen Blutkreislauf I zuführen zu können. Während das Substituat zugeführt wird, ist das Absperrorgan 18A geöffnet und das Absperrorgan 18B geschlossen.

Die Blutbehandlungsvorrichtung verfügt noch über weitere Absperrorgane und Bypass-Leitungen sowie sonstige Komponenten, die aber der Übersichtlichkeit halber nicht dargestellt sind.

Die Leitungsabschnitte des Dialysierflüssigkeitssystems II, über die steriles Substituat aus der zweiten Kammer 15B des zweiten Sterilfilters 15 dem extrakorporalen Blutkreislauf I zugeführt werden, stellen die Substituatsstrecke 17 dar, die von Verunreinigungen, insbesondere der Bildung eines Biofilms, freizuhalten ist. Die Substituatstrecke umfasst daher sämtliche Leitungen oder Leitungsabschnitte, die das Volumen einschließen, das stromab des Sterilfilters liegt, beispielsweise die Verbindungsleitung 17A und den Leitungsabschnitt der Bypassleitung 17B stromauf des Substituatports 17C.

Die Blutbehandlungsvorrichtungen werden nach der Montage überprüft. Anschließend wird das Dialysierflüssigkeitssystem II mit einer Flüssigkeit vollständig befüllt, die ein Reinigungs- und Desinfektionsmittel enthält. Dadurch sind alle Leitungsabschnitte des Dialysierflüssigkeitssystems befüllt. Zu diesem Zeitpunkt ist im Allgemeinen die Substituatleitung 17D nicht an den Substituatport 17C angeschlossen.

Für den anschließenden Transport und die Lagerung der Blutbehandlungsvorrichtung wird das erfindungsgemäße Verfahren unter Verwendung der erfindungsgemäßen Vorrichtung durchgeführt.

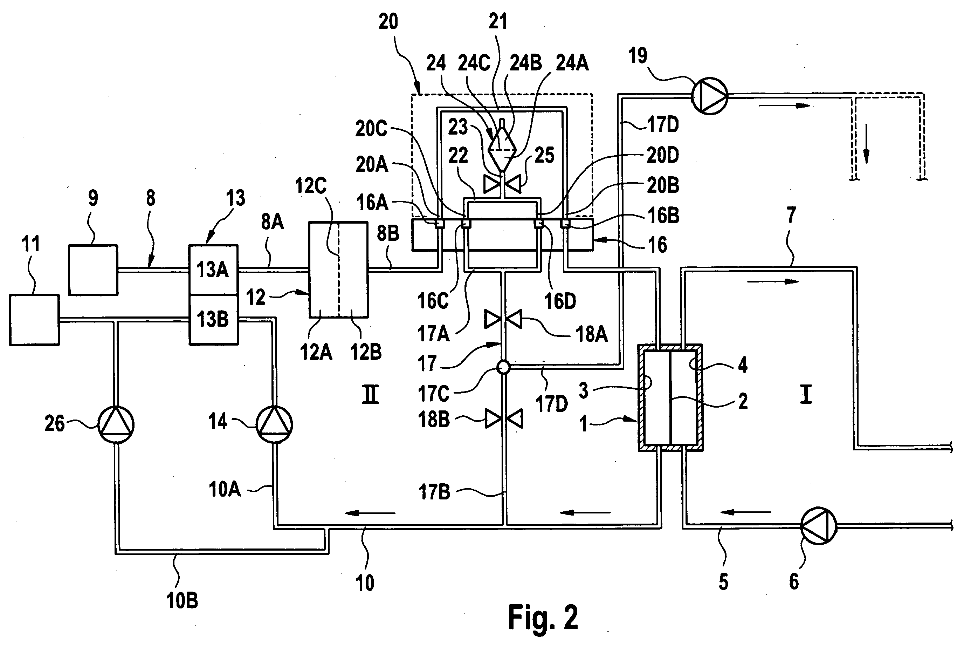

Der erste und zweite Sterilfilter 12, 15 werden entfernt. Dabei wird der erste Sterilfilter 12 in bekannter Weise durch ein bekanntes Kurzschlussstück ersetzt. Der zweite Sterilfilter 15 wird durch die erfindungsgemäße Vorrichtung ersetzt, die nachfolgend unter Bezugnahme auf Fig. 2 beschrieben wird.

Die erfindungsgemäße Vorrichtung 20 zur Durchführung des Konservierungsverfahrens weist wie der zweite Sterilfilter 15 ein erstes Anschlussstück 20A, ein zweites Anschlussstück 20B, ein drittes Anschlussstück 20C und ein viertes Anschlussstück 20D auf, die mit den geräteseitigen Anschlussstücken 16A bis 16D verbunden werden können. Die Anschlussstücke können wieder als passend in Buchsen einsteckbare Stecker ausgebildet sein.

Bei einer bevorzugten Ausführungsform ist das erste und dritte Anschlussstück 20A, 20C als gemeinsamer Stecker ausgebildet, während das zweite und vierte Anschlussstück 20B, 20D als zweiter Stecker ausgebildet ist. Sämtliche Anschlussstücke können aber auch voneinander getrennt sein.

Das erste und zweite Anschlussstück 20A, 20B sind über eine erste Verbindungsleitung 21 und das dritte und vierte Anschlussstück 20C, 20D über eine zweite Verbindungsleitung 22 miteinander verbunden. Die erste und zweite Verbindungsleitung 21, 22 können Schlauchleitungen sein. Von der zweiten Verbindungsleitung 22 zweigt eine dritte Leitung 23 ab, die von einem Sterilfilter 24 verschlossen ist. Der Sterilfilter 24 weist eine erste Kammer 24A und eine zweite Kammer 24B auf, die von einer semipermeablen Membran 24C getrennt sind. Die erste Kammer 24A des Sterilfilters 24 ist mit der dritten Leitung 22, insbesondere Schlauchleitung, verbunden. Zum Abklemmen der Schlauchleitung 23 ist eine Schlauchklemme 25 vorgesehen.

Die erfindungsgemäße Vorrichtung bildet vorzugsweise eine Einheit, die sich leicht und sicher an der Halterung 16 anstelle des Sterilfilters 15 befestigen lässt.

Wenn die erfindungsgemäße Vorrichtung 20 an der Blutbehandlungsvorrichtung angeschlossen ist, wird eine Strömungsverbindung zwischen der Dialysierflüssigkeitsquelle 9 und der Dialysierflüssigkeitskammer 3 hergestellt, während die Substituatsstrecke 17 von dem übrigen Teil des Dialysierflüssigkeitssystems abgetrennt ist. Die Flüssigkeit, die sich in der von dem übrigen Teil des Dialysierflüssigkeitssystems entkoppelten Substituatsstrecke 17 befindet, wird nunmehr abgezogen, so dass die Substituatstrecke frei von Flüssigkeit ist. Hierzu wird das Absperrorgan 25 an der erfindungsgemäßen Vorrichtung geöffnet, so dass zur Entlüftung des von der Substituatsstrecke 17 umschlossenen Volumens Luft in die Substituatsstrecke gelangen kann. Die Flüssigkeit kann beispielsweise in den Ablauf 11 geleitet werden, wobei beispielsweise die Pumpen 14 und 26 betrieben werden können. Anschließend wird das Absperrorgan 25 wieder geschlossen, und der übrige Teil des Dialysierflüssigkeitssystems wird vollständig mit einer Flüssigkeit befüllt, die ein Frostschutzmittel enthält. In diesem Zustand verbleibt die Blutbehandlungsvorrichtung bis zum Aufstellen im Dialysezentrum.

Bei Inbetriebnahme der Dialysevorrichtung werden neue Sterilfilter 12 und 15 eingesetzt. Daraufhin erfolgt der obligatorische Reinigungs- und Desinfektionszyklus. Eventuell eingebrachte Bakterien werden durch die unmittelbare Reinigung und Desinfektion entweder abgetötet oder ausgespült. Somit kann der Erstfiltereinsatz und alle Folgewechsel als aseptisches Verfahren eingestuft werden, die den keimfreien Zustand der Substituatsstrecke auf Dauer garantieren.

Es hat sich in Versuchen gezeigt, dass sich in der nicht mit Frostschutzmittel befüllen Substituatsstrecke auch nach einer längeren Lagerung nicht ein Biofilm bilden kann, der sich nicht sicher und rasch mit einem herkömmlichen Reinigungs- und Desinfektionsmaßnahmen entfernen ließe.

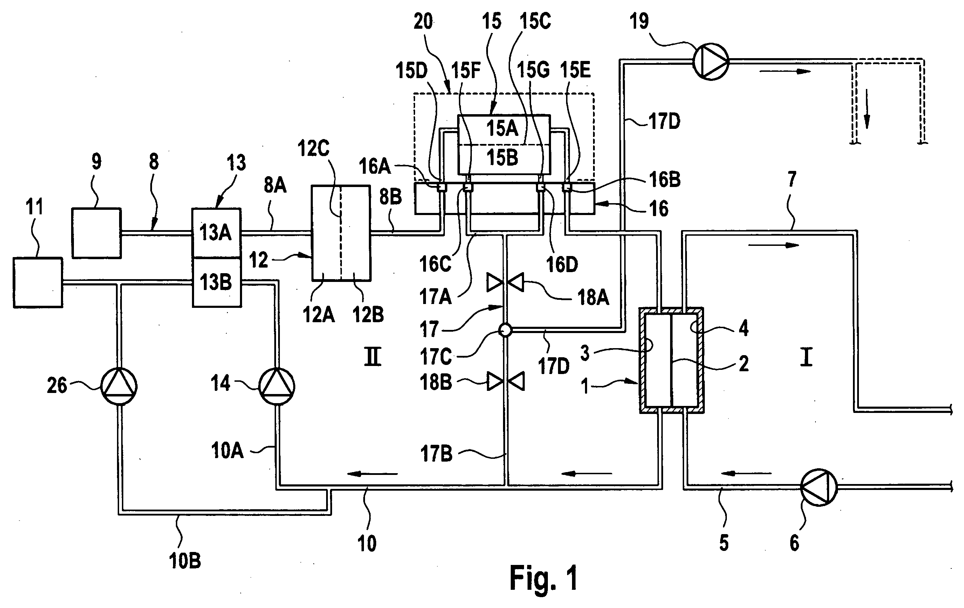

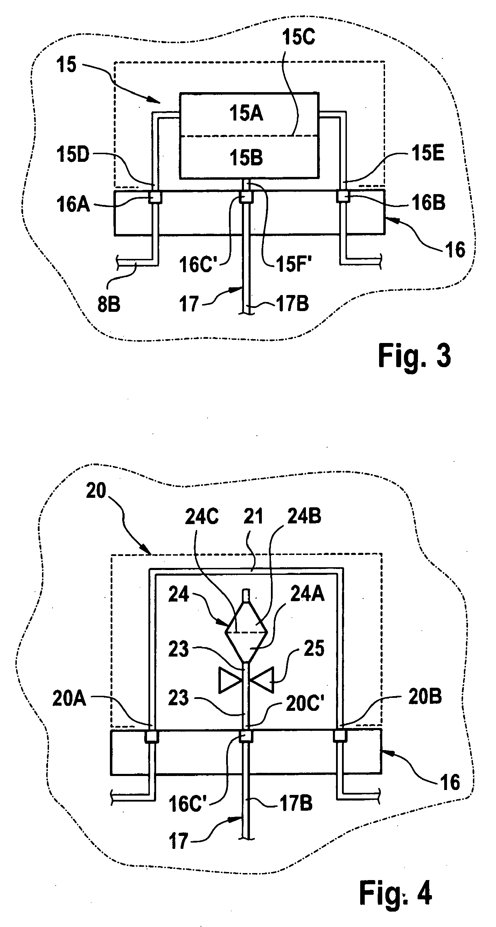

Nachfolgend wird eine alternative Ausführungsform der Blutbehandlungsvorrichtung und der erfindungsgemäßen Vorrichtung zum Anschluss an die Blutbehandlungsvorrichtung unter Bezugnahme auf die Figuren 3 und 4 beschrieben, die sich von der unter Bezugnahme auf die Figuren 1 und 2 beschriebenen Ausführungsform nur dadurch unterscheidet, dass die Blutbehandlungsvorrichtung und die erfindungsgemäße Vorrichtung nur über drei Anschlussstücke verfügen. Die einander entsprechenden Teile sind in den Figuren 3 und 4 mit den gleichen Bezugszeichen wie in den Figuren 1 und 2 versehen. Anstelle der beiden filterseitigen Anschlussstücke 15F und 15G (Fig. 1) weist die zweite Kammer 15B des zweiten Sterilfilters 15 nur ein Anschlussstück 15F' auf (Fig. 3), und anstelle der beiden geräteseitigen Anschlussstücke 16C und 16D (Fig. 1) weist die Blutbehandlungsvorrichtung nur ein Anschlussstücke 16C' auf (Fig. 3), wobei das dritte filterseitige Anschlussstück 15F' an das dritte geräteseitige Anschlussstück 16C' angeschlossen wird (Fig. 3). Eine Verbindungsleitung zum Verbinden von zwei geräteseitigen Anschlussstücken ist hier nicht erforderlich. Die Bypassleitung 17B der Substituatstrecke 17 ist daher direkt mit dem dritten geräteseitigen Anschlussstück 16C'verbunden. Die erste Kammer 24A des Sterilfilters 24 ist über die Verbindungsleitung 23 direkt mit dem dritten Anschlussstück 20C' der erfindungsgemäßen Vorrichtung 20 verbunden (Fig. 4), so dass die Substituatstrecke 17 steril verschlossen ist (Fig. 4), wenn die erfindungsgemäße Vorrichtung an die Blutbehandlungsvorrichtung angeschlossen ist.

## Patentansprüche

1. Vorrichtung zur Durchführung eines Verfahrens zur Konservierung einer extrakorporalen Blutbehandlungsvorrichtung, die ein Dialysierflüssigkeitssystem mit einer Einrichtung zum Anschluss eines Sterilfilters aufweist, welche ein erstes und zweites geräteseitiges Anschlussstück zum Anschluss eines ersten und zweiten filterseitigen Anschlussstücks eines Sterilfilters und ein drittes geräteseitiges Anschlussstück zum Anschluss eines dritten filterseitigen Anschlussstücks des Sterilfilters aufweist,
**dadurch gekennzeichnet, dass**
die Vorrichtung (20) ein erstes Anschlussstück (20A) zum Anschluss an das erste geräteseitige Anschlussstück und ein zweites Anschlussstück (20B) zum Anschluss an das zweite geräteseitige Anschlussstück der Blutbehandlungsvorrichtung sowie ein drittes Anschlussstück (20C') zum Anschluss an das dritte geräteseitige Anschlussstück der Blutbehandlungsvorrichtung aufweist, wobei
das erste und zweite Anschlussstück (20A, 20B) mit einer ersten Verbindungsleitung (21) miteinander verbunden sind und das dritte Anschlussstück (20C') steril verschlossen ist, und
eine Entlüftungseinrichtung (23, 24, 25) vorgesehen ist.

2. Vorrichtung zur Durchführung eines Verfahrens zur Konservierung einer extrakorporalen Blutbehandlungsvorrichtung, die ein Dialysierflüssigkeitssystem mit einer Einrichtung zum Anschluss eines Sterilfilters aufweist, welche ein erstes und zweites geräteseitiges Anschlussstück zum Anschluss eines ersten und zweiten filterseitigen Anschlussstücks eines Sterilfilters und ein drittes und viertes geräteseitiges Anschlussstück zum Anschluss eines dritten und vierten filterseitigen Anschlussstücks des Sterilfilters aufweist,
**dadurch gekennzeichnet, dass**
die Vorrichtung (20) ein erstes Anschlussstück (20A) zum Anschluss an das erste geräteseitige Anschlussstück und ein zweites Anschlussstück (20B) zum Anschluss an das zweite geräteseitige Anschlussstück der Blutbehandlungsvorrichtung sowie ein drittes Anschlussstück (20C) zum Anschluss an das dritte geräteseitige Anschlussstück und ein viertes Anschlussstück (20D) zum Anschluss an das vierte geräteseitige Anschlussstück der Blutbehandlungsvorrichtung aufweist, wobei
das erste und zweite Anschlussstück (20A, 20B) mit einer ersten Verbindungsleitung (21) miteinander verbunden sind und das dritte und vierte Anschlussstück (20C, 20D) mit einer zweiten Verbindungsleitung (22) miteinander verbunden sind, und
eine Entlüftungseinrichtung (23, 24, 25) vorgesehen ist.

3. Vorrichtung nach Anspruch 1, **dadurch gekennzeichnet, dass** die Entlüftungseinrichtung (23, 24, 25) einen durch eine Membran (24C) in eine erste und eine zweite Kammer (24A, 24B) unterteilten Sterilfilter (24) aufweist, wobei die erste Kammer (24A) mit dem dritten Anschlussstück (20C') verbunden ist.

4. Vorrichtung nach Anspruch 3, **dadurch gekennzeichnet, dass** die erste Kammer (24A) mit dem dritten Anschlussstück (20C') über eine Verbindungsleitung (23), insbesondere eine Schlauchleitung, verbunden ist, an der ein Absperrorgan (25) vorgesehen ist.

5. Vorrichtung nach Anspruch 2, **dadurch gekennzeichnet, dass** die Entlüftungseinrichtung (23, 24, 25) einen durch eine Membran (24C) in eine erste und eine zweite Kammer (24A, 24B) unterteilten Sterilfilter (24) aufweist, wobei die erste Kammer (24A) mit der das dritte und vierte Anschlussstück (20C, 20D) verbindenden Verbindungsleitung (22), insbesondere eine Schlauchleitung, verbunden ist.

6. Vorrichtung nach Anspruch 5, **dadurch gekennzeichnet, dass** die erste Kammer (24A) mit der das dritte und vierte Anschlussstück (20C, 20D) verbindenden Verbindungsleitung (22) über eine Verbindungsleitung (23), insbesondere eine Schlauchleitung, verbunden ist, an der ein Absperrorgan (25) vorgesehen ist.

7. Vorrichtung nach einem der Ansprüche 1 bis 6, **dadurch gekennzeichnet, dass** das erste und/oder zweite und/oder dritte und/oder vierte Anschlussstück (20A, 20B, 20C, 20D) als Stecker ausgebildet sind.

8. Vorrichtung nach Anspruch 7, **dadurch gekennzeichnet, dass** das erste und dritte Anschlussstück (20A, 20C) als ein gemeinsamer erster Stecker und das zweite und vierte Anschlussstück (20B, 20D) als ein gemeinsamer zweiter Stecker ausgebildet sind.

9. Extrakorporale Blutbehandlungsvorrichtung mit einem Dialysierflüssigkeitssystem (II), das eine Einrichtung (16) zum Anschluss eines Sterilfilters aufweist, welche ein erstes und zweites geräteseitiges Anschlussstück (16A, 16B) zum Anschluss eines ersten und zweiten filterseitigen Anschlussstücks des Sterilfilters (15) und ein drittes geräteseitiges Anschlussstück (16C') zum Anschluss eines dritten filterseitigen Anschlussstücks des Sterilfilters (15) aufweist, und mit einer
Vorrichtung (20) zur Durchführung eines Verfahrens zur Konservierung der extrakorporalen Blutbehandlungsvorrichtung nach Anspruch 1,
**dadurch gekennzeichnet, dass**
das erste filterseitige Anschlussstück (20A) an dem ersten geräteseitigen Anschlussstück (16A) und das zweite filterseitige Anschlussstück (20B) an dem zweiten geräteseitigen Anschlussstück (16B) sowie das dritte filterseitige Anschlussstück (20C') an dem dritten geräteseitigen Anschlussstück (16C') angeschlossen ist.

10. Blutbehandlungsvorrichtung nach Anspruch 9, **dadurch gekennzeichnet, dass** die Einrichtung zum Anschluss des Sterilfilters (15) als Halteeinrichtung (16) für den Sterilfilter ausgebildet ist, wobei das erste und zweite und dritte geräteseitige Anschlussstück (20A, 20B, 20C') Bestandteil der Halteeinrichtung sind.

11. Extrakorporale Blutbehandlungsvorrichtung mit einem Dialysierflüssigkeitssystem (II), das eine Einrichtung (16) zum Anschluss eines Sterilfilters aufweist, welche ein erstes und zweites geräteseitiges Anschlussstück (16A, 16B) zum Anschluss eines ersten und zweiten filterseitige Anschlussstücks des Sterilfilters (15) und ein drittes und viertes geräteseitiges Anschlussstück (16C, 16D) zum Anschluss eines dritten und vierten filterseitigen Anschlussstücks des Sterilfilters (15) aufweist, und mit einer
Vorrichtung (20) zur Durchführung eines Verfahrens zur Konservierung der extrakorporalen Blutbehandlungsvorrichtung nach Anspruch 2,
**dadurch gekennzeichnet, dass**
das erste filterseitige Anschlussstück (20A) an dem ersten geräteseitigen Anschlussstück (16A) und das zweite filterseitige Anschlussstück (20B) an dem zweiten geräteseitigen Anschlussstück (16B) sowie das dritte filterseitige Anschlussstück (20C) an dem dritten geräteseitigen Anschlussstück (16C) und das vierte filterseitige Anschlussstück (20D) an dem vierten geräteseitigen Anschlussstück (16D) angeschlossen ist.

12. Blutbehandlungsvorrichtung nach Anspruch 11, **dadurch gekennzeichnet, dass** die Einrichtung zum Anschluss des Sterilfilters (15) als Halteeinrichtung (16) für den Sterilfilter ausgebildet ist, wobei das erste und zweite sowie dritte und vierte geräteseitige Anschlussstück (20A, 20B, 20C, 20D) Bestandteil der Halteeinrichtung sind.

13. Verfahren zur Konservierung einer extrakorporalen Blutbehandlungsvorrichtung mit einem extrakorporalen Blutkreislauf und einem Dialysierflüssigkeitssystem, wobei das Dialysierflüssigkeitssystem aufweist:
eine zu einem Dialysator führende Dialysierflüssigkeitszuführleitung, die einen ersten Abschnitt aufweist, an den der Einlass der einen Kammer eines Sterilfilters anschließbar ist, und einen zweiten Abschnitt aufweist, an den der Auslass der einen Kammer eines Sterilfilters anschließbar ist, so dass die eine Kammer des Sterilfilters in die Dialysierflüssigkeitszuführleitung geschaltet werden kann,
eine von dem Dialysator abgehende Dialysierflüssigkeitsabführleitung und
eine zu dem extrakorporalen Blutkreislauf führende Substituatstrecke; die an einem Ende an die andere Kammer des Sterilfilters anschließbar ist,
mit folgenden Verfahrensschritten:
Abtrennen des an den ersten und zweiten Abschnitt der Dialysierflüssigkeitszuführleitung sowie an die Substituatstrecke angeschlossenen Sterilfilters,
Herstellen einer Strömungsverbindung zwischen dem an den Einlass der einen Kammer des Sterilfilter anzuschließenden Ende des ersten Abschnittes der Dialysierflüssigkeitszuführleitung und dem an den Auslass der einen Kammer des Sterilfilters anzuschließenden Ende des zweiten Abschnitts der Dialysierflüssigkeitszuführleitung, so dass die Flüssigkeit durch die Dialysierflüssigkeitszuführleitung unter Umgehung des Sterilfilters in den Dialysator strömen kann,
Steriles Verschließen des an den Sterilfilter anzuschließenden Endes der Substituatstrecke,
Ableiten von in der Substituatstrecke befindlicher Flüssigkeit.

14. Verfahren nach Anspruch 13, **dadurch gekennzeichnet, dass** das Dialysierflüssigkeitssystem mit Ausnahme der Substituatstrecke mit einer Flüssigkeit zum Schutz vor Frost befüllt wird

15. Verfahren nach Anspruch 13 oder 14, **dadurch gekennzeichnet, dass** die in der Substituatstrecke befindliche Flüssigkeit in einen Ablauf geleitet wird.

16. Verfahren nach einem der Ansprüche 13 bis 15, **dadurch gekennzeichnet, dass** zur Inbetriebnahme der Blutbehandlungsvorrichtung nach der Konservierung
das an den Sterilfilter anzuschließende Ende des ersten Abschnittes der Dialysierflüssigkeitszuführleitung und das an den Sterilfilter anzuschließenden Ende des zweiten Abschnitts der Dialysierflüssigkeitszuführleitung mit der einen Kammer des Sterilfilters verbunden wird, so dass eine Flüssigkeit durch die Dialysierflüssigkeitszuführleitung durch den Sterilfilter in den Dialysator strömen kann, und
dass das an den Sterilfilter anzuschließende Ende der Substituatstrecke an die andere Kammer des Sterilfilter angeschlossen wird, und
dass das Dialysierflüssigkeitssystem zur Reinigung oder Desinfektion mit einer Spüllösung gespült wird.

## Claims

1. Device for carrying out a method for conserving an extracorporeal blood treatment device which has a dialysis liquid system with a means for attachment of a sterile filter, which has a first and second appliance-side attachment piece for attachment of a first and second filter-side attachment piece of a sterile filter, and a third appliance-side attachment piece for attachment of a third filter-side attachment piece of the sterile filter,
**characterized in that**
the device (20) has a first attachment piece (20A) for attachment to the first appliance-side attachment piece, and a second attachment piece (20B) for attachment to the second appliance-side attachment piece of the blood treatment device, and also a third attachment piece (20C') for attachment to the third appliance-side attachment piece of the blood treatment device, wherein
the first and second attachment pieces (20A, 20B) are connected to each other by a first connection line (21), and the third attachment piece (20C') is closed in a sterile manner, and
a venting means (23, 24, 25) is provided.

2. Device for carrying out a method for conserving an extracorporeal blood treatment device which has a dialysis liquid system with a means for attachment of a sterile filter, which has a first and second appliance-side attachment piece for attachment of a first and second filter-side attachment piece of a sterile filter, and a third and fourth appliance-side attachment piece for attachment of a third and fourth filter-side attachment piece of the sterile filter,
**characterized in that**
the device (20) has a first attachment piece (20A) for attachment to the first appliance-side attachment piece, and a second attachment piece (20B) for attachment to the second appliance-side attachment piece of the blood treatment device, and also a third attachment piece (20C) for attachment to the third appliance-side attachment piece, and a fourth attachment piece (20D) for attachment to the fourth appliance-side attachment piece of the blood treatment device, wherein
the first and second attachment pieces (20A, 20B) are connected to each other by a first connection line (21), and the third and fourth attachment pieces (20C, 20D) are connected to each other by a second connection line (22), and
a venting means (23, 24, 25) is provided.

3. Device according to Claim 1, **characterized in that** the venting means (23, 24, 25) has a sterile filter (24) divided by a membrane (24C) into a first chamber (24A) and a second chamber (24B), wherein the first chamber (24A) is connected to the third attachment piece (20C').

4. Device according to Claim 3, **characterized in that** the first chamber (24A) is connected to the third attachment piece (20C') by a connection line (23), in particular a hose line, on which a shut-off element (25) is provided.

5. Device according to Claim 2, **characterized in that** the venting means (23, 24, 25) has a sterile filter (24) divided by a membrane (24C) into a first chamber (24A) and a second chamber (24B), wherein the first chamber (24A) is connected to the connection line (22), in particular a hose line, connecting the third and fourth attachment pieces (20C, 20D).

6. Device according to Claim 5, **characterized in that** the first chamber (24A) is connected to the connection line (22), connecting the third and fourth attachment pieces (20C, 20D), via a connection line (23), in particular a hose line, on which a shut-off element (25) is provided.

7. Device according to one of Claims 1 to 6, **characterized in that** the first and/or second and/or third and/or fourth attachment pieces (20A, 20B, 20C, 20D) are designed as plugs.

8. Device according to Claim 7, **characterized in that** the first and third attachment pieces (20A, 20C) are designed as a common first plug, and the second and fourth attachment pieces (20B, 20D) are designed as a common second plug.

9. Extracorporeal blood treatment device with a dialysis liquid system (II) having a means (16) for attachment of a sterile filter, which has a first and second appliance-side attachment piece (16A, 16B) for attachment of a first and second filter-side attachment piece of the sterile filter (15), and a third appliance-side attachment piece (16C') for attachment of a third filter-side attachment piece of the sterile filter (15), and with a
device (20) for carrying out a method for conserving the extracorporeal blood treatment device according to Claim 1,
**characterized in that**
the first filter-side attachment piece (20A) is attached to the first appliance-side attachment piece (16A), the second filter-side attachment piece (20B) is attached to the second appliance-side attachment piece (16B), and the third filter-side attachment piece (20C') is attached to the third appliance-side attachment piece (16C').

10. Blood treatment device according to Claim 9, **characterized in that** the means for attachment of the sterile filter (15) is designed as a holding means (16) for the sterile filter, wherein the first, second and third appliance-side attachment pieces (20A, 20B, 20C') are part of the holding means.

11. Extracorporeal blood treatment device with a dialysis liquid system (II) having a means (16) for attachment of a sterile filter, which has a first and second appliance-side attachment piece (16A, 16B) for attachment of a first and second filter-side attachment piece of the sterile filter (15), and a third and fourth appliance-side attachment piece (16C, 16D) for attachment of a third and fourth filter-side attachment piece of the sterile filter (15), and with a
device (20) for carrying out a method for conserving the extracorporeal blood treatment device according to Claim 2,
**characterized in that**
the first filter-side attachment piece (20A) is attached to the first appliance-side attachment piece (16A), the second filter-side attachment piece (20B) is attached to the second appliance-side attachment piece (16B), the third filter-side attachment piece (20C) is attached to the third appliance-side attachment piece (16C), and the fourth filter-side attachment piece (20D) is attached to the fourth appliance-side attachment piece (16D).

12. Blood treatment device according to Claim 11, **characterized in that** the means for attachment of the sterile filter (15) is designed as a holding means (16) for the sterile filter, wherein the first, second, third and fourth appliance-side attachment pieces (20A, 20B, 20C, 20D) are part of the holding means.

13. Method for conserving an extracorporeal blood treatment device with an extracorporeal blood circuit and a dialysis liquid system,
wherein the dialysis liquid system has:
a dialysis liquid delivery line which leads to a dialyser and has a first section, to which the inlet of one chamber of a sterile filter can be attached, and a second section, to which the outlet of said one chamber of a sterile filter can be attached, such that said one chamber of the sterile filter can be coupled into the dialysis liquid delivery line,
a dialysis liquid removal line leading away from the dialyser, and
a substituate segment, which leads to the extracorporeal blood circuit and which can be attached at one end to the other chamber of the sterile filter, with the following method steps:
separation of the sterile filter attached to the first and second sections of the dialysis liquid delivery line and to the substituate segment,
establishment of a flow connection between the end of the first section of the dialysis liquid delivery line, to be attached to the inlet of one chamber of the sterile filter, and the end of the second section of the dialysis liquid delivery line, to be attached to the outlet of said one chamber of the sterile filter, such that the liquid can flow through the dialysis liquid delivery line into the dialyser, bypassing the sterile filter,
sterile closure of the end of the substituate segment to be attached to the sterile filter,
drainage of liquid located in the substituate segment.

14. Method according to Claim 13, **characterized in that** the dialysis liquid system, except for the substituate segment, is filled with a liquid that protects against freezing.

15. Method according to Claim 13 or 14, **characterized in that** the liquid located in the substituate segment is conveyed into an outflow.

16. Method according to one of Claims 13 to 15, **characterized in that**, in order to put the blood treatment device into operation after the conservation,
the end of the first section of the dialysis liquid delivery line, to be attached to the sterile filter, and the end of the second section of the dialysis liquid delivery line, to be attached to the sterile filter, are connected to one chamber of the sterile filter, such that a liquid can flow through the dialysis liquid delivery line and through the sterile filter into the dialyser, and
**in that** the end of the substituate segment to be attached to the sterile filter is attached to the other chamber of the sterile filter, and
**in that** the dialysis liquid system is cleaned or disinfected by flushing it with a flushing solution.

## Revendications

1. Dispositif pour la mise en oeuvre d'un procédé de conservation d'un dispositif de traitement de sang extracorporel, qui comprend un système de liquide de dialyse avec un arrangement pour le raccordement d'un filtre stérile, lequel comprend un premier et un deuxième raccord côté appareil pour le raccordement d'un premier et d'un deuxième raccord côté filtre d'un filtre stérile et un troisième raccord côté appareil pour le raccordement d'un troisième raccord côté filtre du filtre stérile,
**caractérisé en ce que**
le dispositif (20) comprend un premier raccord (20A) pour le raccordement au premier raccord côté appareil et un deuxième raccord (20B) pour le raccordement au deuxième raccord côté appareil du dispositif de traitement de sang, ainsi qu'un troisième raccord (20C') pour le raccordement au troisième raccord côté appareil du dispositif de traitement de sang, dans lequel
le premier et le deuxième raccord (20A, 20B) sont reliés entre eux par une première conduite de liaison (21) et le troisième raccord (20C') est scellé de manière stérile, et
est prévu un arrangement pour la ventilation (23, 24, 25).

2. Dispositif de mise en oeuvre d'un procédé de conservation d'un dispositif de traitement de sang extracorporel, qui comprend un système de liquide de dialyse avec un arrangement pour le raccordement d'un filtre stérile, lequel comprend un premier et un deuxième raccord côté appareil pour le raccordement d'un premier et d'un deuxième raccord côté filtre d'un filtre stérile et un troisième et un quatrième raccord côté appareil pour le raccordement d'un troisième et d'un quatrième raccord côté filtre du filtre stérile, **caractérisé en ce que**
le dispositif (20) comprend un premier raccord (20A) pour le raccordement au premier raccord côté appareil et un deuxième raccord (20B) pour le raccordement au deuxième raccord côté appareil du dispositif de traitement de sang, ainsi qu'un troisième raccord (20C) pour le raccordement au troisième raccord côté appareil et un quatrième raccord (20D) pour le raccordement au quatrième raccord côté appareil du dispositif de traitement de sang, dans lequel
le premier et le deuxième raccord (20A, 20B) sont reliés entre eux par une première conduite de liaison (21) et le troisième et le quatrième raccord (20C, 20D) sont reliés entre eux par une deuxième conduite de liaison (22), et
est prévu un arrangement pour la ventilation (23, 24, 25).

3. Dispositif selon la revendication 1, **caractérisé en ce que** l'arrangement pour la ventilation (23, 24, 25) comprend un filtre stérile (24) divisé par une membrane (24C) en une première et une deuxième chambre (24A, 24B), dans lequel la première chambre (24A) est reliée au troisième raccord (20C').

4. Dispositif selon la revendication 3, **caractérisé en ce que** la première chambre (24A) est reliée au troisième raccord (20C') par l'intermédiaire d'une conduite de liaison (23), plus particulièrement une conduite flexible, au niveau de laquelle un organe d'arrêt (25) est prévu.

5. Dispositif selon la revendication 2, **caractérisé en ce que** l'arrangement pour la ventilation (23, 24, 25) comprend un filtre stérile (24) divisé par une membrane (24C) en une première et une deuxième chambre (24A, 24B), dans lequel la première chambre (24A) est reliée à la conduite de liaison (22) reliant le troisième et le quatrième raccord (20C, 20D), plus particulièrement une conduite flexible.

6. Dispositif selon la revendication 5, **caractérisé en ce que** la première chambre (24A) est reliée à la conduite de liaison (22) reliant le troisième et le quatrième raccord (20C, 20D) par l'intermédiaire d'une conduite de liaison (23), plus particulièrement une conduite flexible, au niveau de laquelle un organe d'arrêt est prévu (25).

7. Dispositif selon l'une des revendications 1 à 6, **caractérisé en ce que** le premier et/ou le deuxième et/ou le troisième et/ou le quatrième raccord (20A, 20B, 20C, 20D) sont configurés comme des connecteurs.

8. Dispositif selon la revendication 7, **caractérisé en ce que** le premier et le troisième raccord (20A, 20C) sont conçus comme un premier connecteur commun, et le deuxième et le quatrième raccord (20B, 20D) sont configurés comme un deuxième connecteur commun.

9. Dispositif de traitement de sang extracorporel avec un système de liquide de dialyse (II), qui comprend un arrangement (16) pour le raccordement d'un filtre stérile, lequel comprend un premier et un deuxième raccord côté appareil (16A, 16B) pour le raccordement d'un premier et d'un deuxième raccord côté filtre du filtre stérile (15) et un troisième raccord côté appareil (16C') pour le raccordement d'un troisième raccord côté filtre du filtre stérile (15), et avec un
dispositif (20) pour la mise en oeuvre d'un procédé de conservation du dispositif de traitement de sang extracorporel selon la revendication 1,
**caractérisé en ce que**
le premier raccord côté filtre (20A) est raccordé au premier raccord côté appareil (16A), le deuxième raccord côté filtre (20B) est raccordé au deuxième raccord côté appareil (16B), et le troisième raccord côté filtre (20C') est raccordé au troisième raccord côté appareil (16C').

10. Dispositif de traitement de sang extracorporel selon la revendication 9, **caractérisé en ce que** l'arrangement pour le raccordement du filtre stérile (15) est configuré comme un arrangement de maintien (16) pour le filtre stérile, dans lequel le premier, le deuxième et le troisième raccord côté appareil (20A, 20B, 20C') font partie intégrante de l'arrangement de maintien.

11. Dispositif de traitement de sang extracorporel avec un système de liquide de dialyse (II), qui comprend un arrangement (16) pour le raccordement d'un filtre stérile, lequel comprend un premier et un deuxième raccord côté appareil (16A, 16B) pour le raccordement d'un premier et d'un deuxième raccord côté filtre du filtre stérile (15) et un troisième et un quatrième raccord côté appareil (16C, 16D) pour le raccordement d'un troisième et d'un quatrième raccord côté filtre du filtre stérile (15) et avec un dispositif (20) pour la mise en oeuvre d'un procédé de conservation du dispositif de traitement de sang extracorporel selon la revendication 2,
**caractérisé en ce que**
le premier raccord côté filtre (20A) est raccordé au premier raccord côté appareil (16A) et le deuxième raccord côté filtre (20B) est raccordé au deuxième raccord côté appareil (16B), le troisième raccord côté filtre (20C) est raccordé au troisième raccord côté appareil (16C) et le quatrième raccord côté filtre (20D) est raccordé au quatrième raccord côté appareil (16D).

12. Dispositif de traitement de sang selon la revendication 11, **caractérisé en ce que** l'arrangement pour le raccordement du filtre stérile (15) est configuré comme un arrangement de maintien (16) pour le filtre stérile, dans lequel le premier et le deuxième ainsi que le troisième et le quatrième raccord côté appareil (20A, 20B, 20C, 20D) font partie intégrante de l'arrangement de maintien.

13. Procédé de conservation d'un dispositif de traitement de sang extracorporel avec un circuit sanguin extracorporel et un système de liquide de dialyse,
dans lequel le système de liquide de dialyse comprend :
une conduite d'alimentation en liquide de dialyse conduisant vers un dialyseur, qui comprend une première portion à laquelle l'entrée d'une chambre d'un filtre stérile est raccordable, et une deuxième portion à laquelle la sortie d'une chambre d'un filtre stérile est raccordable, de sorte qu'une chambre du filtre stérile puisse être branchée à la conduite d'alimentation en liquide de dialyse,
une conduite de liquide de dialyse sortant du dialyseur et
un trajet de substitution conduisant au circuit sanguin extracorporel, qui est raccordable au niveau d'une extrémité à l'autre chambre du filtre stérile,
avec les étapes suivantes de procédé :
séparation du filtre stérile raccordé à la première et deuxième portion de la conduite d'alimentation en liquide de dialyse ainsi qu'au trajet de substitution,
établissement d'une liaison d'écoulement entre l'extrémité de la première portion de la conduite d'alimentation en liquide de dialyse à raccorder à l'entrée d'une chambre du filtre stérile et l'extrémité de la deuxième portion de la conduite d'alimentation en liquide de dialyse à raccorder à la sortie d'une chambre du filtre stérile, de sorte que le liquide puisse s'écouler à travers la conduite d'alimentation en liquide de dialyse vers le dialyseur en contournant le filtre stérile,
scellement étanche de l'extrémité du trajet de substitution à raccorder au filtre,
dérivation du liquide se trouvant dans le trajet de substitution.

14. Procédé selon la revendication 13, **caractérisé en ce que** le système de liquide de dialyse est rempli par un liquide anti-gel, à l'exception du trajet de substitution.

15. Procédé selon la revendication 13 ou 14; **caractérisé en ce que** le liquide se trouvant dans le trajet de substitution est conduit vers une évacuation.

16. Procédé selon l'une des revendications 13 à 15, **caractérisé en ce que**, pour la mise en service du dispositif de traitement de sang, après la conservation,
l'extrémité de la première portion de la conduite d'alimentation en liquide de dialyse à raccorder au filtre stérile et l'extrémité de la deuxième portion de la conduite d'alimentation en liquide de dialyse à raccorder au filtre stérile sont reliées à une chambre du filtre stérile, de sorte qu'un liquide puisse s'écouler à travers la conduite d'alimentation en liquide de dialyse, à travers le filtre stérile, vers le dialyseur, et
**en ce que** l'extrémité du trajet de substitution à raccorder au filtre stérile est raccordée à l'autre chambre du filtre stérile, et
**en ce que** le système de liquide de dialyse est rincé pour le nettoyage ou la désinfection avec une solution de rinçage.
